Europäisches Patentamt

European Patent Office

Office européen des brevets

(11) Veröffentlichungsnummer : **0 005 509**
**B1**

(12) **EUROPÄISCHE PATENTSCHRIFT**

(45) Veröffentlichungstag der Patentschrift :
**15.04.81**

(21) Anmeldenummer : **79101422.8**

(22) Anmeldetag : **10.05.79**

(51) Int. Cl.³ : **C 07 D 307/68**

(54) Verfahren zur Herstellung von O-Methyl-N-cyclohexyl-2,5-dimethylfuran-3-hydroxamsäure.

(30) Priorität : **13.05.78 DE 2821087**

(43) Veröffentlichungstag der Anmeldung :
**28.11.79 (Patentblatt 79/24)**

(45) Bekanntmachung des Hinweises auf die Patenterteilung : **15.04.81 Patentblatt 81/15**

(84) Benannte Vertragsstaaten :
**AT BE CH DE FR GB IT LU NL SE**

(56) Entgegenhaltungen :
**DE - A - 2 455 082**
**US - A - 3 778 512**

(73) Patentinhaber : **BASF Aktiengesellschaft**
**Carl-Bosch-Strasse 38**
**D-6700 Ludwigshafen (DE)**

(72) Erfinder : **Linhart, Friedrich, Dr.**
**Leisberg 61**
**D-6900 Heidelberg (DE)**
Erfinder : **Girgensohn, Bjoern, Dr.**
**Neckarpromenade 38**
**D-6800 Mannheim (DE)**
Erfinder : **Schulte, Wolfgang, Dr.**
**Barbarossastrasse 9**
**D-6733 Hassloch (DE)**
Erfinder : **Mueller, Hans Richard, Dr.**
**An der Tuchbleiche 9**
**D-6712 Bobenheim-Roxheim (DE)**

Imprimerie Jouve, 17, rue du Louvre, 75001 Paris, France

Verfahren zur Herstellung von 0-Methyl-N-cyclohexyl-2,5-dimethylfuran-3-hydroxamsäure

Die vorliegende Erfindung betrifft ein Verfahren zur Herstellung von 0-Methyl-N-cyclohexyl-2,5-dimethylfuran-3-hydroxamsäure durch Umsetzung von N-Cyclohexylhydroxylamin mit 2,5-Dimethylfuran-3-carbonsäurechlorid und Methylierung der Hydroxamsäure.

Es ist bekannt (DE-OS 24 55 082), 0-Methyl-N-cyclohexyl-2,5-dimethylfuran-3-hydroxamsäure durch Methylierung von N-Cyclohexyl-2,5-dimethylfuran-3-hydroxamsäure mit Dimethylsulfat in einem Natronlauge-Dichlormethangemisch herzustellen und anschließend zu destillieren.
Die N-Cyclohexyl-2,5-dimethylfuran-3-hydoxamsäure wird durch Umsetzung von N-Cyclohexylhydroxylaminhydrochlorid mit 2,5-Dimethylfuran-3-carbonsäurechlorid im Lösungsmittel Benzol unter Verwendung der Hilfsbase Triäthylamin hergestellt und als Niederschlag isoliert.

Es hat sich gezeigt, daß dieses zweistufige Verfahren für eine technische Durchführung aus mehreren Gründen nicht gut geeignet ist. So muß z.B. das Endprodukt destilliert werden. Geschieht dies nicht sehr sorgfältig, dann sind Verunreinigungen im Endprodukt enthalten. Andererseits beginnt sich die Substanz bei ihrem Siedepunkt $Kp_{0,3}$ 133 bis 135 °C bereits merklich zu zersetzen, so daß ein Verzicht auf die Destillation in der Praxis unumgänglich scheint.

Ein zweiter Nachteil des bekannten Verfahrens besteht darin, daß man die als Zwischenprodukt anfallende N-cyclohexyl-2,5-dimethylfuran-3-hydroxamsäure aus einem organischen brennbaren Lösungsmittel absaugen muß, was, insbesondere beim Entleeren der Nutsche, besonderer Sicherheitsmaßnahmen bedarf, um Brände und Explosionen zu vermeiden. Weitere nicht so schwerwiegende Nachteile der bekannten Methode bestehen darin, daß man für den ersten Reaktionsschritt Triäthylamin als Hilfsbase verwenden muß, was bei seinem unangenehmen Geruch und seiner schleimhautreizenden Wirkung in der Praxis nicht ohne besondere Vorsichtsmaßnahmen möglich ist, und daß die Methylierung unter Verwendung eines organischen Lösungsmittels durchgeführt wird.

Es wurde nun gefunden, daß man alle diese Schwierigkeiten vermeidet und ein reineres Endprodukt in besserer Ausbeute als beim bekannten Verfahren erhält, wenn man N-Cyclohexylhydroxylamin mit 2,5-Dimethylfuran-3-carbonsäurechlorid in Gegenwart von Wasser, einem flüssigen Kohlenwasserstoff und einer anorganischen alkalischen Verbindung umsetzt, die so erhaltene N-Cyclohexeyl-2,5-dimethylfuran-3-hydroxamsäure durch Behandlung der Reaktionsmitschung mit einer wäßrigen Alkalihydroxidlösung in die wäßrige Lösung ihres Alkalisalzes überführt, danach die Verbindung in dieser Lösung methyliert und die so hergestellte 0-Methyl-N-cyclohexyl-2,5-dimethylfuran-3-hydroxamsäure mit einem mit Wasser nicht mischbaren organischen Lösungsmittel aus der wäßrigen Lösung abtrennt. Beispielsweise wird das N-Cyclohexylhydroxylamin oder sein Hydrochlorid in einem Wasser-Toluol (oder Xylol)-Gemisch bei pH 8 bis 10 mit 2,5-Dimethylfuran-3-carbonsäurechlorid umgesetzt, wobei dieser pH-Wert durch Zugabe einer anorganischen alkalischen Verbindung vor oder während der Umsetzung eingehalten wird ; das Reaktionsgemisch wird beispielsweise mit wäßriger Natronlauge oder Kalilauge behandelt, die wäßrige Lösung mit Toluol gewaschen und mit Dimethylsulfat verrührt, eventuell überschüssiges Dimethylsulfat wird mit Ammoniak zerstört, das ölige Reaktionsprodukt in einem mit Wasser nicht mischbaren organischen Lösungsmittel, vorzugsweise Toluol, gelöst, die organische Lösung mit Natronlauge und Wasser gewaschen und eingedampft, wobei 0-Methyl-N-cyclohexyl-2,5-dimethylfuran-3-hydroxamsäure in einer Reinheit von 99 % (Gew. %) zurückbleibt.

Bei dem ersten Schritt des Verfahrens kann man so vorgehen, daß man N-Cyclohexylhydroxylamin zunächst, zweckmäßig im Reaktionsgefäß in Anwesenheit von Toluol, aus einer wäßrigen Lösung seines Hydrochlorids mit Hilfe einer anorganischen Base, z.B. eines Alkali- oder Erdalkalimetallhydroxids oder eines Alkali- oder Erdalkalimetallcarbonats oder -hydrogencarbonats in Freiheit setzt und dann das 2,5-Dimethylfuran-3-carbonsäurechlorid bei pH 8 bis 10 zusetzt, wobei man zur Einhaltung dieses pH entweder vor Beginn der Umsetzung eine Puffersubstanz oder ein Puffergemisch z.B. das Hydrogencarbonat eines Alkali- oder Erdalkalimetalls, der Lösung zusetzt, oder aber während der Umsetzung eine starke Base, z.B. ein Alkalimetallhydroxid in dem Maße zugibt, in dem das pH unter den Wert 8 abzusinken droht. Die Umsetzung wird beispielsweise im Temperaturbereich von 0 bis 100 °C, vorzugsweise 10 bis 50 °C, insbesondere 15 bis 35 °C, in einem Zeitraum von 1 bis 5 Stunden, vorzugsweise 1,5 Stunden, in Gegenwart von Toluol, verschiedenen Xylolen, Cyclohexan, Hexan, Heptan, Octan oder Gemischen dieser Flüssigkeiten durchgeführt. Wird die Umsetzung ohne pH-Regelung durchgeführt dann kann man als Basen beispielsweise Natriumhydrogencarbonat, Kaliumhydrogencarbonat, Magnesiumcarbonat oder Calciumcarbonat verwenden. Wird die Umsetzung dagegen mit pH-Regelung durchgeführt dann kann man als Basen beispielsweise Natriumhydroxid, Kaliumhydroxid, Calciumhydroxid, Bariumhydroxid, Natriumcarbonat, Kaliumcarbonat, Natriumhydrogencarbonat oder Kaliumhydrogencarbonat verwenden. Bei der Umsetzung beträgt das Molverhältnis Cyclohexylhydroxylamin zu 2,5-Dimethylfuran-3-carbonsäurechlorid beispielsweise 0,9 zu 1 bis 1,1 zu 1 Mol. Das Molverhältnis anorganische Base zu 2,5-Dimethylfuran-3-carbonsäurechlorid beträgt beispielsweise 1 bis 2 oder mehr zu 1 Mol. Die Umsetzung kann beispielsweise bei Normaldruck oder leichtem Über- oder Unterdruck (z.b.10 bar) durchgeführt werden. Man kann aber auch so verfahren, daß man N-Cyclohexylhydroxylaminhydrochlorid als wäßrige Lösung in ein Gemisch aus Toluol, Wasser und eine der oben bezeichneten Basen einlaufen läßt und anschließend die Umsetzung bei pH 8 bis 10 durchführt. Die Methylierung wird beispielsweise bei einer Temperatur von 0 bis 80 °C, vorzugsweise 10 bis 30 °C, in einer Zeit von 2 bis 15 Stunden in Gegenwart von Toluol, Xylolen, höheren Alkylaromaten oder ganz allgemein aliphatischen oder cycloaliphatischen Kohlenwasserstoffen oder chlorierten Kohlenwasserstoffen z.B. Dichlormethan oder Dichloräthan und Natriumhydroxid oder Kaliumhydroxid durchgeführt. Beispielsweise verwendet man bei der Methylierung mit Dimethylsulfat einen Überschuß von 3 bis 50 Mol % Dimethylsulfat vorzugsweise 10 bis 30 Mol % bezogen auf das Säurechlorid oder das Hydroxylamin und 50 bis 200 Mol % Überschuß an Alkalihydroxid vorzugsweise 100 Mol % bezogen auf die Ausgangsmaterialien. Die Umsetzung kann beispielsweise bei Normaldruck oder leichtem Unter- oder Überdruck (z.B. 10 bar) durchgeführt werden. Im Anschluß an den zweiten Reaktionsschritt, die Methylierung, empfiehlt es sich, für die Abrennung des Produkts aus seiner wäßrigen Emulsion ein organisches mit Wasser nicht mischbares Lösungsmittel zu verwenden, welches leichter als Wasser ist, so daß die wäßrigen Waschlösungen abgelassen werden können und die organische Produktlösung im Reaktionsgefäß verbleibt. Besonders eignen sich hierfür Lösungsmittel, in welchem die Substanz weiter verarbeitet wird z.B. Toluol, so daß man auf das Eindampfen der Lösung verzichten kann und eine gebrauchsfertige Lösung von 0-Methyl-N-cyclohexyl-2,5-dimethylfuran-3-hydroxamsäure erhält. Unter der Voraussetzung, daß solche Lösungsmittel nicht mit einem der Reaktionspartner reagieren, kann man sie auch bereits bei der Methylierungsreaktion der Reaktionsmischung zusetzen.

Die Vorteile des erfindungsgemäßen Verfahrens bestehen zusammengefaßt darin, daß man :

a) das Zwischenprodukt N-Cyclohexyl-2,5-dimethylfuran-3-hydroxamsäure nicht zu isolieren und dazu keine besonderen Schutzvorkehrungen braucht ;

b) das Endprodukt ohne Destillation in 99 %-iger Reinheit, in besserer Ausbeute und falls gewünscht bereits in einem für die Weiterverarbeitung geeigneten Lösungsmittel erhält ;

c) die Ausgangssubstanzen bei einem durch Zugabe von anorganischen Basen aufrecht erhaltenen pH-Wert von 8 bis 10 in Wasser umsetzt.

Es ist überraschend, daß es gelingt, ohne Isolierung der N-Cyclohexyl-2,5-dimethylfuran-3-hydroxamsäure und ohne Destillation des Endproduktes ein reines Endprodukt in guter Ausbeute zu erhalten.

Die folgenden Beispiele sollen die Vorzüge des erfindungsgemäßen Verfahrens demonstrieren :

Beispiel 1

Zu einer Mischung aus 90 Gewichtsteilen Wasser, 50 Gewichtsteilen Toluol und 45,5 Gewichtsteilen N-Cyclohexylhydroxylaminhydrochlorid gibt man bis zur Neutralisation 24 Gewichtsteile 50 %-ige (Gew. %) wäßrige Natronlauge. Anschließend gibt man 28 Gewichtsteile Natriumhydrogencarbonat zu. Bei 20 bis 30 °C werden unter gutem Rühren im Laufe von 1 1/2 Stunden 50 Gewichtsteile 91 %-iges 2,5-Dimethylfuran-3-carbonsäurechlorid zugesetzt. Nach halbstündigem Nachrühren gibt man 52,5 Gewichtsteile 50 %-ige Natronlauge und 400 Gewichtsteile Wasser zu und mischt kräftig. Die Wasserphase wird abgetrennt und bei 20 bis 30 °C im Laufe von 2 bis 3 Stunden mit 50 Gewichtsteilen Dimethylsulfat versetzt. Nach dem Rühren über Nacht gibt man 10 Gewichtsteile konzentrierte Ammoniaklösung zu und rührt 2 Stunden. Anschließend verrührt man mit 70 Gewichtsteilen Toluol, trennt die Toluolphase ab, wäscht sie i einmal mit 40 Gewichtsteilen 10 %-iger Natronlauge und einmal mit 40 Gewichtsteilen Wasser. Die Lösung wird im Vakuum mit Hilfe eines Dünnschichtverdampfers vom Lösungsmittel befreit. Man erhält 55 Gewichtsteile 0-Methyl-N-cyclohexyl-2,5-dimethylfuran-3-hydroxamsäure in 99 %-iger Reinheit.

Beispiel 2

Zu einer Suspension von 168 Gewichtsteilen Natriumhydrogencarbonat in 300 Gewichtsteilen Wasser und 330 Gewichtsteilen Toluol setzt man eine Lösung von 151,5 Gewichtsteilen N-Cyclohexylhydroxylaminhydrochlorid in 250 Gewichtsteilen Wasser. Anschließend gibt man 167 Gewichtsteile 91 %-iges 2,5-Dimethylfuran-3-carbonsäurechlorid zu und rührt 1/2 Stunde nach. Nach Zugabe von 600 Gewichtsteilen 10 %-iger Natronlauge wird kräftig gemischt, die wäßrige Phase abgetrennt und mit 149 Gewichtsteilen Dimethylsulfat 12 Stunden gerührt. Nach anschließender halbstündiger Durchmischung mit 50 Gewichtsteilen konzentrierter Ammoniaklösung wird das ölige Produkt in 250 Gewichtsteilen Toluol gelöst, die Toluollösung mit 10 %-iger Natronlauge und mit Wasser gewaschen und eingedampft. Man erhält 195 Gewichtsteile reine 0-Methyl-N-cyclohexyl-2,5-dimethylfuran-3-hydroxamsäure.

Beispiel 3

115 Gewichtsteile N-Cyclohexylhydroxylamin werden in 330 Gewichtsteilen Toluol und 300 Gewichtsteilen Wasser suspendiert. Der pH-Wert der Mischung wird mit einem pH-Meter über eine Glaselektrode bestimmt. Dann läßt man unter gutem Rühren gleichzeitig, aber auch verschiedene Einlässe, 167 Gewichtsteile 91 %-iges 2,5-Dimethylfuran-3-carbonsäurechlorid und so schnell und soviel 25 %-ige Natronlauge zufließen, daß der pH-Wert zwischen 8 und 10 bleibt. Nach anschließender Zugabe von 600 Gewichtsteilen 10 %-iger Natronlauge wird weiterverfahren, wie in Beispiel 2 beschrieben. Man erhält 203 Gewichtsteile reine 0-Methyl-N-cyclohexyl-2,5-dimethylfuran-3-hydroxamsäure.

**Ansprüche**

1. Verfahren zur Herstellung von 0-Methyl-N-cyclohexyl-2,5-dimethylfuran-3-hydroxamsäure durch Umsetzung von N-Cyclohexylhydroxylamin oder seinem Hydrochlorid mit 2,5-Dimethylfuran-3-carbonsäurechlorid in Gegenwart eines organischen Lösungsmittels und einer alkalischen Verbindung und Methylierung der Hydroxamsäure in Gegenwart von Wasser und Alkali, *dadurch gekennzeichnet*, daß man N-Cyclohexylhydroxylamin mit 2,5-Dimethylfuran-3-carbonsäurechlorid in Gegenwart von Wasser, eines flüssigen Kohlenwasserstoffs und einer anorganischen alkalischen Verbindung umsetzt, die so erhaltene N-Cyclohexyl-2,5-dimethylfuran-3-hydroxamsäure durch Behandlung der Reaktionsmischung mit einer wäßrigen Alkalihydroxidlösung in die wäßrige Lösung ihres Alkalisalzes überführt, danach die Verbindung in dieser Lösung methyliert und die so hergestellte 0-Methyl-N-cyclohexyl-2,5-dimethylfuran-3-hydroxamsäure mit einem mit Wasser nicht mischbaren organischen Lösungsmittel aus der wäßrigen Lösung abtrennt.

2. Verfahren gemäß Anspruch 1, *dadurch gekennzeichnet*, daß man das 2,5-Dimethylfuran-3-carbonsäurechlorid mit dem N-Cyclohexylhydroxylamin bei pH 8 bis 10 reagieren läßt.

3. Verfahren gemäß Anspruch 1, *dadurch gekennzeichnet*, daß man die Methylierung mit Dimethylsulfat in Gegenwart eines für die Weiterverwendung des Endproduktes geeigneten organischen Lösungsmittels durchführt.


## Claims

1. A process for the preparation of 0-methyl-N-cyclohexyl-2,5-dimethylfuran-3-hydroxamic acid by reacting N-cyclohexylhydroxylamine or its hydrochloride with 2,5-dimethylfuran-3-carboxylic acid chloride in the presence of an organic solvent and of an alkaline compound and methylating the hydroxamic acid in the presence of water and alkali, *characterized in that* N-cyclohexylhydroxylamine is reacted with 2,5-dimethylfuran-3-carboxylic acid chloride in the presence of water, a liquid hydrocarbon and an inorganic alkaline compound, the resulting N-cyclohexyl-2,5-dimethylfuran-3-hydroxamic acid is converted, by treating the reaction mixture with an aqueous alkali metal hydroxide solution, to an aqueous solution of its alkali metal salt, the compound is then methylated in this solution and the 0-methyl-N-cyclohexyl-2,5-dimethylfuran-3-hydroxamic acid thus prepared is isolated from the aqueous solution by means of a water-immiscible organic solvent.

2. A process as claimed in claim 1, *characterized in that* 2,5-dimethylfuran-3-carboxylic acid chloride is reacted with N-cyclohexyl-hydroxylamine at pH 8-10.

3. A process as claimed in claim 1, *characterized in that* the methylation is carried out with dimethyl sulfate in the presence of an organic solvent suitable for the further use of the end product.


## Revendications

1. Procédé de préparation d'acide 0-méthyl-N-cyclohexyl-2,5-diméthylfuran-3-hydroxamique par réaction de N-cyclohexylhydroxylamine ou son chlorure avec un chlorure d'acide 2,5-diméthylfuran-3-carboxylique en présence d'un solvant organique et d'un composé alcalin et méthylation de l'acide hydroxamique en présence d'eau et d'alcali, caractérisé par le fait que l'on fait réagir de la N-cyclohexylhydroxylamine avec un chlorure d'acide 2,5-diméthylfuran-3-carboxylique en présence d'eau, d'un hydrocarbure liquide et d'un composé alcalin inorganique, on transfère l'acide N-cyclohexyl-2,5-diméthylfuran-3-hydroxamique dans la solution aqueuse de son sel alcalin par traitement du mélange de réaction avec une solution aqueuse d'hydroxyde alcalin, puis on méthyle le composé dans cette solution et on sépare de la solution aqueuse l'acide 0-méthyl-N-cyclohexyl-2,5-diméthylfuran-3-hydroxamique ainsi préparé, avec un solvant organique non miscible avec l'eau.

2. Procédé selon la revendication 1, dans lequel on fait agir le chlorure d'acide 2,5-diméthylfuran-3-carboxylique avec la N-cyclohexylhydroxylamine à un pH de 8 à 10.

3. Procédé selon la revendication 1, dans lequel on effectue la méthylation avec du sulfate de diméthyle en présence d'un solvant organique convenant à l'utilisation ultérieure du produit final.